# EUROPEAN PATENT APPLICATION

(11) **EP 3 552 542 A1**
(43) Date of publication of application: **16.10.2019**
(21) Application number: 18190675.1
(22) Date of filing: 24.08.2018
(51) Int. Cl.: A61B 5/0428, A61B 5/0408, A61B 5/00

(54) **ELECTROCARDIOGRAPHIC MEASUREMENT METHOD AND ELECTROCARDIOGRAPHIC MEASUREMENT DEVICE**

(30) Priority: 10.04.2018 JP 2018075181
(71) Applicant: Simplex Quantum Inc., Tokyo 108-0074 (JP)
(72) Inventor: MIKAMI, Yoshihiro, Tokyo, 108-0074 (JP)
(74) Representative: Perronace, Andrea

(57) **Abstract**

The present disclosure provides an electrocardiographic measurement method, including: preparing a bar body to be gripped by a hand of a subject of an electrocardiographic measurement; providing a first and second electrode with a space in an axial direction of the bar body and providing a ground electrode with a space in a circumferential direction of the bar body from at least one of the first and second electrodes; connecting an electrocardiogram measuring circuit between the first and second electrodes, and measuring an electrocardiogram signal of the subject by an electrocardiogram measuring circuit while the subject grips the bar body with right and left hands at positions of the first and second electrodes so as to bring the right and left hands into contact with the first and second electrodes, respectively, and to bring the ground electrode into contact with one of the right and left hands.

## Description

### CROSS-REFERENCE TO RELATED APPLICATION

This application claims priority pursuant to 35 U.S.C. §119 from Japanese Patent Application No. 2018-075181, filed on April 10, 2018, the entire disclosure of which is incorporated herein by reference.

### BACKGROUND

### Technical Field

The present disclosure relates to an electrocardiographic (ECG) measurement method and an electrocardiographic (ECG) measurement device.

### Related Art

An electrocardiograph is a measuring device that measures electric potentials generated by myocardium to cause heartbeats. Changes in these electric potentials can be extracted as electrocardiogram (ECG) signals. ECG signals can be utilized for detection of various heart diseases such as angina pectoris in their early stage and for observation or the like of the condition of autonomic nerves. Thus, the electrocardiograph can be considered as one of basic biological information measuring devices.

ECG signals show extremely weak changes in electric potentials. For this reason, a LEAD XII ECG system is used for precisely measuring ECG signals with ten electrodes in total placed on the chest, wrist and ankle. However, a LEAD I method in which ECG signals are simply measured with attachment of two or three electrodes at positions interposing the heart therebetween is often used as well, the attachment being a so-called LEAD I. This LEAD I method can be easily performed and frequently measure ECG signals for long periods, thus detecting changes of the ECG signals that are unable to be obtained by less-frequent measurements with the LEAD XII ECG system. Accordingly, it is not unusual to obtain valuable information that contributes to early detection of lesion or the like through the LEAD I method.

The followings are proposed as examples of an ECG measurement device that adopts an ECG measurement using such a LEAD I method. An ECG signal detecting device in WO2010/113354 adopts a configuration in which at least a pair of electrodes is provided on a surface of a display window arranged in a case, and right and left thumbs are brought into contact with the electrodes, respectively. Moreover, a blood pressure measurement device in JP2013-226189A has a configuration in which electrodes for detecting ECG signals are provided on a case with a peripheral surface of the case to be held with both hands so that the ECG signals can be measured while the case is being held with both hands. In addition, WO2016/181808 describes a configuration including a substantially spheroidal body and performing an ECG measurement by bringing one hand into contact with a first electrocardiographic electrode while the body is gripped with the one hand, and bringing the other hand into contact with a second electrocardiographic electrode provided in the body.

As described above, ECG signals show extremely weak changes in electric potentials. Thus, when a measurement with LEAD I method is performed, for example, by bringing a finger into contact with a measuring electrode, acquisition of ECG signals may be difficult due to reduction of a signal-to-noise ratio caused by external electromagnetic noise. Furthermore, ease of measurement of ECG signals also has individual differences, and it has been known from experience that there are subjects whose ECG signals cannot be measured simply by bringing their fingers into contact with the measuring electrodes. For example, if it is considered that the ECG signals are regarded as one of biological information to be used as data for personal authentication, such a method of simply bringing fingers into contact with the measuring electrodes to measure the ECG signals of LEAD I has concerns about frequent events such that the authentication is impossible due to electromagnetic environment of measuring places or individual characteristics of the subjects.

### SUMMARY

The present disclosure has been made to solve the foregoing and other problems, and an objective of the present disclosure is to provide an ECG measurement method and an ECG measurement device each capable of stably measuring ECG signals of LEAD I with a simple configuration.

An electrocardiographic measurement method according to an aspect of the present disclosure for achieving the foregoing and other objectives, includes preparing a bar body having a thickness capable of being gripped by a hand of a person who is a subject of an electrocardiographic measurement; providing a first electrode and a second electrode for an electrocardiogram signal measurement of the subject with a space in an axial direction of the bar body and providing a ground electrode with a space in a circumferential direction of the bar body from at least one of the first electrode and the second electrode; connecting an electrocardiogram measuring circuit between the first electrode and the second electrode, the electrocardiogram measuring circuit including an amplifier; and measuring an electrocardiogram signal of the subject by the electrocardiogram measuring circuit through the first electrode and the second electrode while the subject grips the bar body at parts where right and left hands come to positions of the first electrode and the second electrode so as to bring the right and left hands of the subject into contact with the first electrode and the second electrode, respectively, and to bring the ground electrode into contact with one of the right and left hands.

Moreover, an electrocardiographic measurement device according to another aspect of the present disclosure, includes a bar body having a thickness capable of being gripped by a hand of a person who is a subject of an electrocardiographic measurement; a first electrode and a second electrode for an electrocardiogram signal measurement of the subject, the first electrode and the second electrode being provided with a space in an axial direction of the bar body; a ground electrode provided with a space in a circumferential direction of the bar body from at least one of the first electrode and the second electrode; and an electrocardiogram measuring circuit connected between the first electrode and the second electrode, the electrocardiogram measuring circuit including an amplifier; an electrocardiogram signal of the subject being measured by the electrocardiogram measuring circuit through the first electrode and the second electrode while the subject grips the bar body at parts where right and left hands come to positions of the first electrode and the second electrode so as to bring the right and left hands of the subject into contact with the first electrode and the second electrode, respectively, and to bring the ground electrode into contact with one of the right and left hands.

In the electrocardiographic measurement method and the electrocardiographic measurement device, the first electrode and the second electrode, and the ground electrode paired with one of the first electrode and the second electrode with a space in a circumferential direction of the bar body may be disposed at positions so as to be covered with the right and left hand of the subject.

Moreover, in the electrocardiographic measurement method and the electrocardiographic measurement device, the bar body may be configured as a cylindrical case housing the electrocardiogram measuring circuit.

Furthermore, in the electrocardiographic measurement method and the electrocardiographic measurement device, the bar body may be a part of a handrail on which a person puts a hand.

An electrocardiographic measurement method according to another aspect of the present disclosure, includes preparing a bar body having a thickness capable of being gripped by a hand of a person who is a subject of an electrocardiographic measurement; providing a first electrode for an electrocardiogram signal measurement of the subject on a circumferential surface of the bar body and providing a ground electrode with a space in a circumferential direction of the bar body from the first electrode; providing a second electrode for an electrocardiogram signal measurement of the subject on one end surface of the bar body in an axial direction; connecting an electrocardiogram measuring circuit between the first electrode and the second electrode, the electrocardiogram measuring circuit including an amplifier; and measuring an electrocardiogram signal of the subject by the electrocardiogram measuring circuit through the first electrode and the second electrode while the subject grips the bar body at a part where one hand comes to a position of the first electrode, and the second electrode is pressed against a chest near a heart of the subject so that the one hand of the subject gripping the bar body is brought into contact with the first electrode and the ground electrode, and the second electrode is brought into contact with the chest of the subject.

This electrocardiographic measurement method may be performed by an electrocardiographic measurement device including a bar body having a thickness capable of being gripped by a hand of a person who is a subject of an electrocardiographic measurement; a first electrode for an electrocardiogram signal measurement of the subject, the first electrode being provided on a circumferential surface of the bar body; a ground electrode provided with a space in a circumferential direction of the bar body from the first electrode; a second electrode for an electrocardiogram signal measurement of the subject, the second electrode being provided on one end surface of the bar body in an axial direction; and an electrocardiogram measuring circuit connected between the first electrode and the second electrode, the electrocardiogram measuring circuit including an amplifier, an electrocardiogram signal of the subject being measured by the electrocardiogram measuring circuit through the first electrode and the second electrode while the subject grips the bar body at a part where one hand comes to a position of the first electrode, and the second electrode is pressed against a chest near a heart of the subject so that the one hand of the subject gripping the bar body is brought into contact with the first electrode and the ground electrode, and the second electrode is brought into contact with the chest of the subject.

According to one aspect of the present disclosure, ECG signals of LEAD I can be stably measured with a simple configuration.

### BRIEF DESCRIPTION OF DRAWINGS

FIG. 1 is a perspective view illustrating a configuration example of an ECG measurement device 1 according to an embodiment of the present disclosure;
FIG. 2 is a schematic side view of the ECG measurement device 1 of FIG. 1;
FIG. 3 is a circuit block diagram illustrating a configuration example of an ECG measuring circuit used in the ECG measurement device 1 of FIG. 1;
FIG. 4 is a schematic diagram illustrating how the ECG measurement device 1 corresponding to FIG. 1 is used;
FIG. 5 is a schematic diagram illustrating how the ECG measurement device 1 corresponding to FIG. 2 is used;
FIG. 6A is a schematic diagram illustrating an example of an ECG waveform measured by a conventional ECG measurement method;
FIG. 6B is a schematic diagram illustrating an example of an ECG waveform measured by an ECG measurement method of the present embodiment;
FIG. 7 is a perspective view illustrating a configuration example of the ECG measurement device 1 according to a modified example of an embodiment of the present disclosure;
FIG. 8 is a circuit block diagram illustrating a periphery of measuring electrodes of the ECG measurement device 1 of FIG. 7; and
FIG. 9 is a schematic diagram illustrating a usage state of the ECG measurement device 1 of FIG. 7.

### DETAILED DESCRIPTION OF THE EMBODIMENTS

A description of the present disclosure based on an embodiment thereof will be made with reference to the drawings.

In describing embodiments illustrated in the drawings, specific terminology is employed for the sake of clarity. However, the disclosure of the present specification is not intended to be limited to the specific terminology so selected and it is to be understood that each specific element includes all technical equivalents that have a similar function, operate in a similar manner, and achieve a similar result.

As used herein, the singular forms "a", "an", and "the" are intended to include the plural forms as well, unless the context clearly indicates otherwise.

Referring to the drawings, wherein like reference numerals designate identical or corresponding parts throughout the several views, embodiments of the present disclosure are described below. Electrocardiographic (ECG) Measurement Device

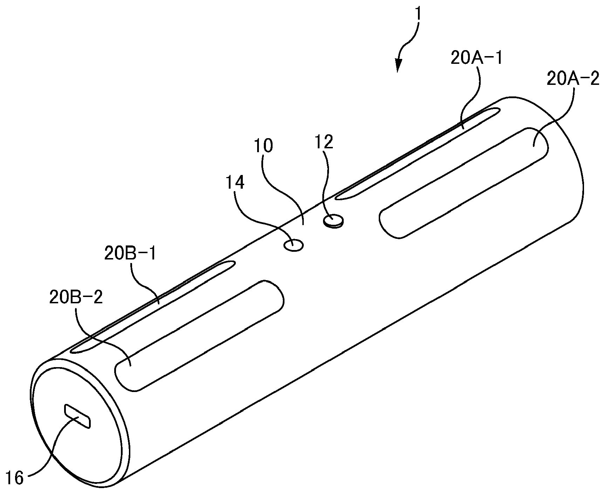

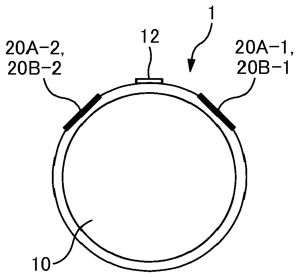

FIG. 1 and FIG. 2 illustrate an example of an ECG measurement device for achieving an ECG measurement method according to an embodiment of the present disclosure. FIG. 1 is a perspective view of an ECG measurement device 1, and FIG. 2 is a side view thereof.

The ECG measurement device 1 includes a cylindrical case 10. The case 10 is produced as a pair of half-split hollow resin components, for example, that is made into a cylindrical shape by being combined. Electric and electronic components such as an ECG measuring circuit described later and a power supply such as a battery are housed inside the case 10. A longitudinal dimension of the case 10 in an axial direction may be set to such an extent that a subject of an ECG measurement can grip the case 10 with both hands aligned, and accordingly the longitudinal dimension may be about 170 mm as an example. An outer diameter of the case 10 may be set to such an extent to provide a proper space between the subject's thumb and opposing four fingers when the subject gently grips the case 10 with his/her hand. Accordingly, the outer diameter may be about 40 mm as an example. A resin material for forming the case 10 is not particularly limited as long as the material offers a comfortable touch when touched with hands, has durability and is harmless to the skin. In consideration that many people's hands touch the case 10, the case 10 may be formed using an antibacterial resin material and may be coated with antibacterial coating. It should be noted that the outline of the cross-section of the case 10 may be formed not only in a circular shape, but also in an elliptical shape, a polygonal shape, or the like. Those shapes give an effect that prevents the case 10 from rolling on the desk or the like.

Measuring electrodes 20A-1 and 20B-1 (first electrode and second electrode) are provided on a circumferential surface of the case 10 with a space in an axial direction of the case 10. Moreover, ground electrodes 20A-2 and 20B-2 are provided so as to respectively correspond to the measuring electrodes 20A-1 and 20B-1 with a space in the circumferential direction of the case 10. In the example of the present embodiment, the measuring electrodes 20A-1 and 20B-1, and the ground electrodes 20A-2 and 20B-2 are configured of metal plates each having an identical shape dimension. As illustrated in FIG. 1, though the metal plates for these electrodes are each formed into an elliptical shape, the metal plates are not limited thereto and may have other shapes such as a rectangular shape. Each of the metal plates for the electrodes preferably has a dimension such that each electrode is hidden within a range of the width of the hand when the subject grips the case 10 with his/her hand. Thus, for example, the dimension of each metal plate in the axial direction of the case 10 may be about 60 mm. In the present embodiment, the width of each of the metal plates for the electrodes is 10 mm. However, this dimension can be also appropriately determined depending on design requirements or the like. Moreover, each metal plate for the electrode is touched by the subject's hand, and thus the metal plate is preferably configured of a conductive metal plate having a corrosion resistance so as not to cause corrosion or the like due to adhesion of sweat. Each electrode can be formed by a conductive material other than metal plates, and it can be considered that, for example, conductive fiber, conductive pastes or the like may be applicable.

A separation distance between the measuring electrodes 20A-1, 20B-1 and the ground electrodes 20A-2, 20B-2 in the circumferential direction of the case 10 may be determined to an appropriate dimension in consideration of connection with an internal circuit or the like.

Although an ECG measurement method of the present embodiment will be described later, it is important for the foregoing ECG measurement device 1 to form the case 10 into a shape dimension capable of being easily gripped and allow the case 10 to have a shape dimension and be positioned such that each electrode is almost hidden by the subject's hand when the subject grips the case 10. From this perspective, when the ECG measurement device 1 is designed, for example, a database configured to contain dimensions of each part of the human body can be appropriately used. As such a database, there is "dimension data of Japanese hands, in 2012, AIST, Makiko KOUCHI" (https://www.dh.aist.go.jp/database/hand/index.html), for example.

As illustrated in FIG. 1 and FIG. 2, a power supply switch 12 and an indicator 14 are provided between the measuring electrode 20A-1 and the ground electrode 20A-2, and the measuring electrode 20B-1 and the ground electrode 20B-2, which are separated in an axial direction. The power supply switch 12 is configured as a push switch for turning on/off the power supply of the ECG measuring circuit described later, and the indicator 14 is configured as an LED lamp indicating a state of the ECG measuring circuit.

A connector 16 is provided on one end surface of the case 10 in the longitudinal direction. In the present embodiment, the connector 16 is configured so as to be capable of connecting a USB cable (USB Type-C), thus being used for charging power source for the ECG measuring circuit and for transmission of ECG data.

It should be noted that the power supply switch 12, the indicator 14 and the connector 16 are not limited to the aspects of the present embodiment, and their installation positions, types, functions and the like can be properly determined. ECG Measuring Circuit

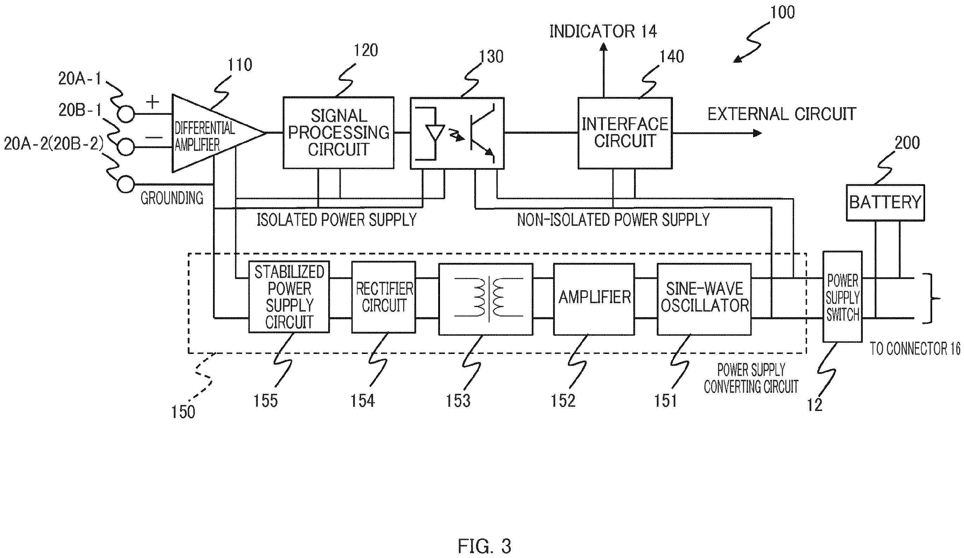

A description will be given regarding the ECG measuring circuit. FIG. 3 is a circuit block diagram illustrating an exemplary configuration of an ECG measuring circuit 100 used in the ECG measurement device 1 of the present embodiment. The ECG measuring circuit 100 in FIG. 3 has a function of amplifying ECG signals of LEAD I detected between the measuring electrodes 20A-1 and 20B-1 and converting the ECG signals into digital signals to be outputted.

The ECG measuring circuit 100 illustrated in FIG. 3 includes the measuring electrodes 20A-1 and 20B-1, the ground electrodes 20A-2 or 20B-2, a differential amplifier 110, a signal processing circuit 120, a photocoupler 130, an interface circuit 140 and a power supply converting circuit 150.

The measuring electrodes 20A-1 and 20B-1 are connected to a non-inverting input and inverting input of the differential amplifier 110. The ground electrodes 20A-2 and 20B-2 are connected to a ground circuit of the ECG measuring circuit 100 and serve to enhance noise resistance performance of the ECG measuring circuit 100. At least either one of the ground electrodes 20A-2 and 20B-2 may be essentially connected to the ground circuit.

ECG analog signals inputted from the measuring electrodes 20A-1 and 20B-1 to the differential amplifier 110 are inputted to the signal processing circuit 120 after being amplified. The signal processing circuit 120 includes a well-known waveform shaping circuit and an analog-to-digital (A/D) converter, and digitizes the ECG analog signals that are outputs of the differential amplifier 110 to convert them into ECG digital signals. The ECG digital signals are delivered to the interface circuit 140 through the photocoupler 130. The ECG digital signals are transmitted from the signal processing circuit 120 side to the interface circuit 140 side as optical signals by the photocoupler 130. This enables electrical isolation between the signal processing circuit 120 and the interface circuit 140.

The interface circuit 140 includes a level conversion circuit or the like as a preceding stage of exporting the ECG digital signals to an external circuit including an arithmetic processor or the like that imports the ECG digital signal as data to process them. Moreover, the interface circuit 140 may be provided with a communication interface circuit for sending the ECG digital signals to an external device such as a smartphone. The communication interface circuit also includes a Near Field Communication (NFC) interface or the like such as Bluetooth (R) . It should be noted that the arithmetic processor included in the present interface circuit 140 can also control a lighting state of the indicator 14 of the ECG measurement device 1.

In this manner, the present ECG measuring circuit 100 can extract the digitized ECG signals of the subject.

A description will be given regarding a power supply system of the ECG measuring circuit 100. The power of the ECG measuring circuit 100 is supplied by the power supply converting circuit 150. The power supply converting circuit 150 illustrated in FIG. 3 includes a sine-wave oscillator 151, an amplifier 152, an isolating transformer 153, a rectifier circuit 154 and a stabilizing power supply circuit 155. The power to the power supply converting circuit 150 is supplied by a battery 200. The battery 200 may be selected from batteries of appropriate type and capacity, and a nickel-hydrogen secondary battery can be given as an example. The power supply switch 12 is provided between the battery 200 and the power supply converting circuit 150. The battery 200 is capable of being connected to an external power supply via the connector 16.

The power supply converting circuit 150 illustrated in a broken line part of FIG. 3 uses an external DC power supply (for example, 5V USB power supply) as an input, supplies non-isolated power using the same ground potential as that of the external DC power supply, and supplies isolated power having a ground potential different from the foregoing ground potential. In the ECG measuring circuit 100 in FIG. 3, isolated power from the secondary side of the isolating transformer 153 is supplied to the differential amplifier 110 connected to the measuring electrodes 20A-1, 20B-1 and the ground electrode 20A-2 or 20B-2, the signal processing circuit 120 and an input section of the photocoupler 130. Non-isolated power from the primary side of the isolating transformer 153 is supplied to an output section of the photocoupler 130 and the interface circuit 140.

In the power supply converting circuit 150, the sine-wave oscillator 151 first generates a sine wave using a non-isolated power supply (an external DC power supply) . The sine-wave signal is inputted to the amplifier 152 to be amplified and its output is inputted to the isolating transformer 153. The output of the isolating transformer 153 is converted into a direct current at the rectifier circuit 154 and supplied to the differential amplifier 110, the signal processing circuit 120 and the input section of the photocoupler 130 as an isolated power supply via the stabilizing power supply circuit 155.

As a sine-wave oscillation circuit used for the sine-wave oscillator 151, a Colpitts circuit, a Hartley circuit or its modified circuit can be used as a circuit using both an inductor and a capacitor, and a Wien bridge circuit, a phase-shift oscillation circuit or its modified circuit can be used as a circuit using a resistor and a capacitor. The sine-wave oscillator 151 described here is a circuit configured for oscillation of a sine wave and is practically allowed to output a waveform slightly deviated from the sine wave due to an adjustment degree of circuit constants or the like. It is sufficient if the signal does not have a waveform significantly deviated from the sine wave like a waveform including a square wave or a spike-shaped peak. However, it may be designed appropriately. Although an oscillation frequency of the sine wave is not particularly limited, from the point of view of practical use, it is considered that the oscillation frequency is set to a value of, for example, about 10 kHz or more and 500 kHz or less.

The sine wave outputted from the sine-wave oscillator 151 is inputted to the isolating transformer 153 after amplification by the amplifier 152. The circuit configuration of this amplifier 152 may be optional, and it is sufficient if power of sufficient magnitude is inputted into the isolating transformer 153 without excessively distorting the waveform of the sine wave to be inputted and causing parasitic oscillation. In this respect, if the output from the sine-wave oscillator 151 is large enough, the amplifier 152 may be eliminated.

The isolating transformer 153 includes a core constituting a magnetic circuit obtained by forming a ferromagnetic ferrite material into a closed shape such as a ring shape, and a primary winding and a secondary winding are wound on the core. The primary winding and the secondary winding are isolated in direct current. The insulation voltage between the primary side and the secondary side is generally about 1000 to 2000 V. It is generally preferable to increase AC voltage by allowing the winding ratio that is a ratio of the number of secondary windings to the number of primary windings to be more than "1".

The rectifier circuit 154 is preferably configured with, for example, a bridge type full-wave rectifier circuit using a Schottky diode and a smoothing circuit connected its output. However, the rectifier circuit 154 may be configured with another circuit having a similar function.

The output of the rectifier circuit 154 is supplied as an isolated power supply after the voltage is stabilized using the stabilizing power supply circuit 155. As the stabilizing power supply circuit 155, an appropriate circuit configuration can be adopted except for a circuit system which causes a problem of noise generation such as a switching regulator.

In the power supply converting circuit 150 of FIG. 3, only the circuit blocks that are essential for the function of the circuit 150 is illustrated. However, a stabilizing power supply circuit other than these circuit blocks may be inserted before the non-isolated power is supplied or the power is supplied to the sine-wave oscillator 151.

When the ECG measuring circuit 100 operates with the primary battery or internal secondary battery and transmits the ECG signals by radio communication such as Bluetooth (R) while being completely isolated from the external DC power supply, the ground electrode 20A-2 or 20B-2 is practically allowed to be eliminated.

However, when the ECG measurement is performed with the external DC power supply connected, power induction noise of 50 Hz or 60 Hz, which is a commercial frequency, enters the isolated power supply side through a capacity between an input and an output of the isolating transformer 153 or a capacity between an input and an output of the photocoupler 130, and accordingly this may cause large noise signal. In this case, the ground electrode 20A-2 or 20B-2 is connected to the ground potential of the isolated power supply to bring fingers into contact with the ground electrode 20A-2 or 20B-2, thus enabling the large noise signal to be greatly reduced.

The ECG measuring circuit 100 of the present embodiment can obtain an effect that the measurement can be performed without concern for risk of electrical shock of the subject even in a period of connecting the ECG measurement device 1 to the external power supply for charging.

### Electrocardiographic (ECG) Measurement Method

A description will be given regarding an ECG measurement method that is one embodiment of the present disclosure. This ECG measurement method can be typically easily achieved by the ECG measurement device 1 described above. However, the method is not limited to a method using the ECG measurement device 1.

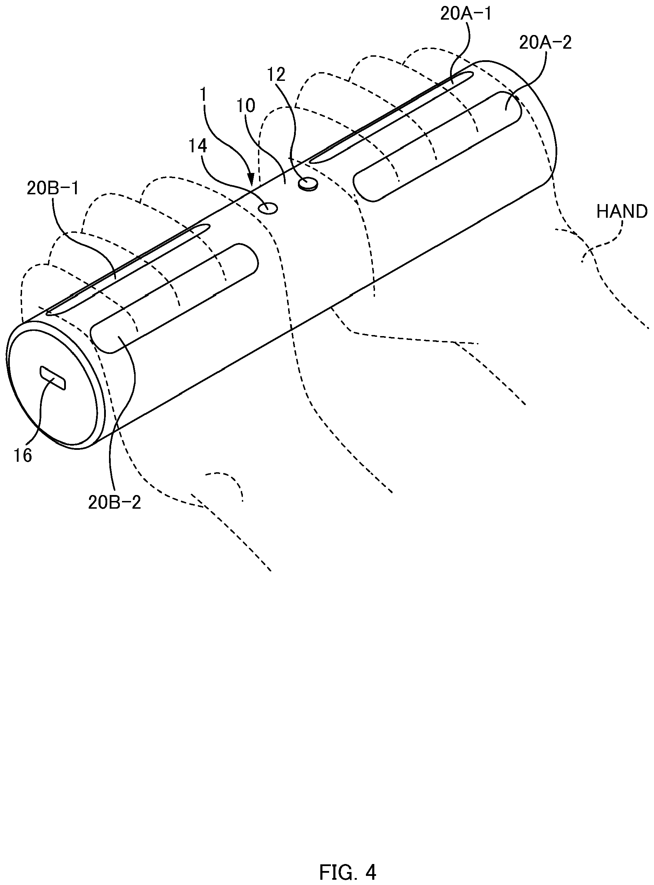

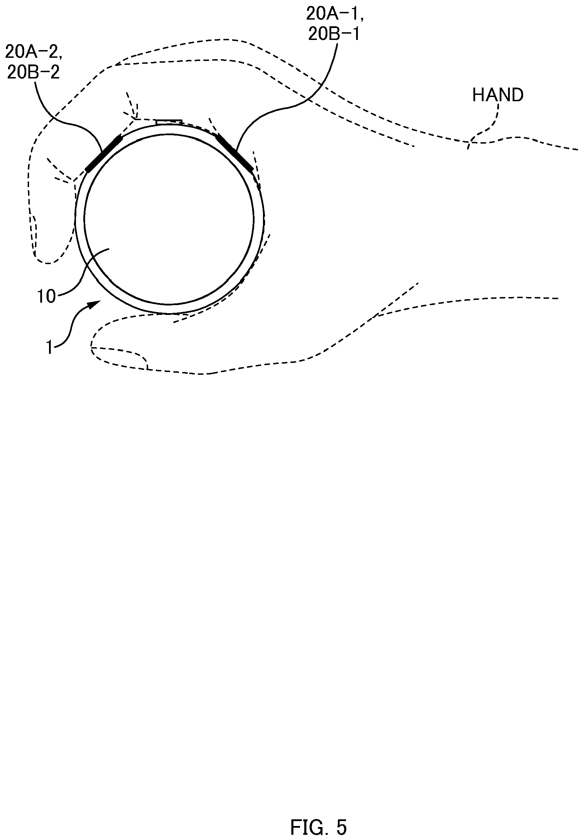

FIG. 4 and FIG. 5 illustrate how the ECG measurement is performed using the ECG measurement device 1 of FIG. 1. When the ECG measurement is performed, the subject grips the case 10 of the ECG measurement device 1 so as to hold the case 10 with the subject's hands so that the thumb and remaining four fingers are placed at respective positions where the measuring electrodes 20A-1 and 20B-1 and the ground electrodes 20A-2 and 20B-2 of the ECG measurement device 1 are provided. Due to the relationship among installation positions and dimensional shapes of the respective electrodes 20A-1 to 20B-2, and an outer diameter of the case 10 of the ECG measurement device 1, the electrodes 20A-1 to 20B-2 are almost or completely hidden by the fingers and palm of the subject (see FIG. 4). This is a state during the ECG measurement using the ECG measurement device 1.

As an example of a measurement procedure, the subject first operates the power supply switch 12 to turn on the ECG measurement device 1. Accordingly, the ECG measuring circuit 100 is set to a standby state for an ECG measurement start, and lights the indicator 14 to send a notice of the standby state.

In this state, the subject grips the case 10 of the ECG measurement device 1 so as to bring the subject's fingers into contact with each of the electrodes 20A-1 to 20B-2 sufficiently as described above. The ECG measuring circuit 100 executes processing of ECG signals received from the measuring electrodes 20A-1 and 20B-1 to generate ECG signal data. Whether or not the generation of the ECG signal data was successful can be notified by the lighting state of the indicator 14. When the subject has learned the end of measurement due to the lighting state of the indicator 14 or elapse of a predetermined time since the subject gripped the ECG measurement device 1 to start the measurement, the subject releases the held ECG measurement device 1 to end the ECG measurement.

### Advantageous Effects According To the Present Electrocardiographic (ECG) Measurement Method

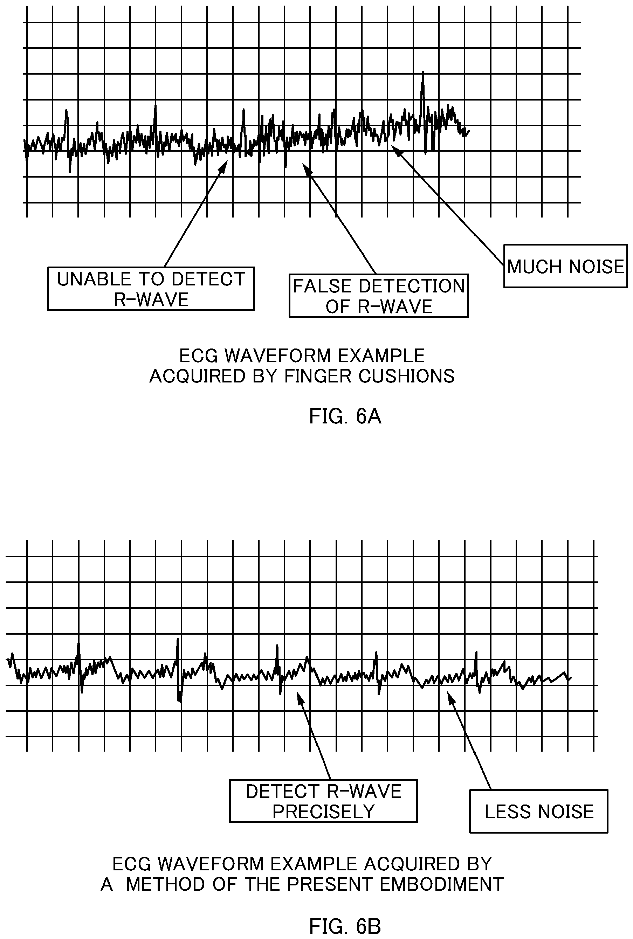

The ECG measurement method described above is capable of significantly enhancing a signal-to-noise ratio at the time of ECG measurement. FIG. 6A illustrates an example of an ECG waveform measured by bringing the finger cushions into contact with the measuring electrodes, and FIG. 6B illustrates an example of an ECG waveform obtained from the ECG measurement performed by the identical subject using the ECG measurement device 1 of the present embodiment. When FIG. 6A is compared with FIG. 6B, it is clear that noise superimposed on the ECG waveform is smaller in FIG. 6B and R-waves characterizing an ECG waveform are more distinctly measured. In this comparative example, the signal-to-noise ratio is improved by about 6 dB in the ECG waveform acquired by the ECG measurement method of the present disclosure as compared with the conventional method.

In this manner, the following can be considered as a reason for the remarkable improvement in the measurement result of the ECG waveform.
- The entire measuring electrodes 20A-1 and 20B-1 are in contact with the fingers and palm of the subject in large areas, thus suppressing contact resistance to be small and stabilizing a contact state. This prepares an environment suitable for detecting weak electric potentials of the ECG signals.
- Gripping the bar body with hands is an action that can be realized naturally by a person. Accordingly, unnecessary strain is not put on muscles of the hands or arms, thus suppressing myoelectric signals that become noise to be small.
- The measurement is performed in a state in which the entire measuring electrodes 20A-1 and 20B-1 are covered with the subject's hands, and accordingly noise reduction is achieved by the subject's hands serving as electromagnetic shields. Modified Example of Electrocardiographic (ECG) Measurement Method

In the embodiments described above, the ECG measurement is performed using the ECG measurement device 1 illustrated in FIG. 1 or the like. As stated above, when the ECG measurement device 1 is gripped with both hands to perform the ECG measurement, a preferable ECG waveform having less noise can be acquired. However, the subject whose one arm or hand has lost its function due to handicap or who has lost one arm or hand due to an accident or the like is not able to perform the measurement using such a method. The present modified example provides a solution to such a subject who is unable to or hardly performs the ECG measurement with both hands.

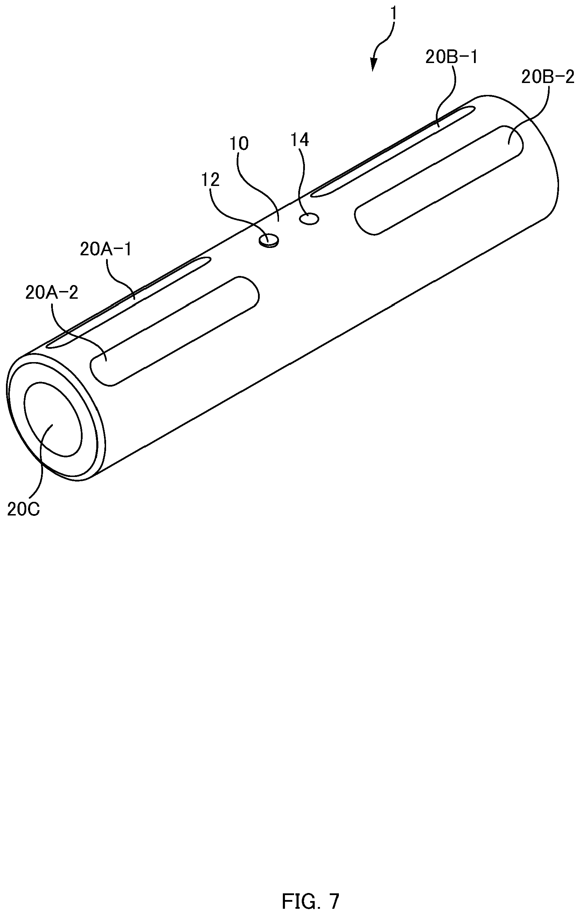

FIG. 7 illustrates a configuration example of the ECG measurement device 1 according to the present modified example. It should be noted that the elements of FIG. 7 corresponding to those of FIG. 1 are denoted by the same reference numerals for simplification.

The ECG measurement device 1 of FIG. 7 differs from the one illustrated in FIG. 1 in that a measuring electrode 20C is provided on an end surface of the measuring electrode 20A-1 side in the axial direction. The measuring electrode 20C is formed of a conductive metal plate or the like in the same manner as the measuring electrodes 20A-1 and 20B-1. In the example of FIG. 7, although the measuring electrode 20C has a circular shape, it may have other shapes such as a rectangle. The measuring electrode 20C is pressed against the subject's chest by the subject having a problem with one hand or arm, instead of the measuring electrode 20A-1 or 20B-1.

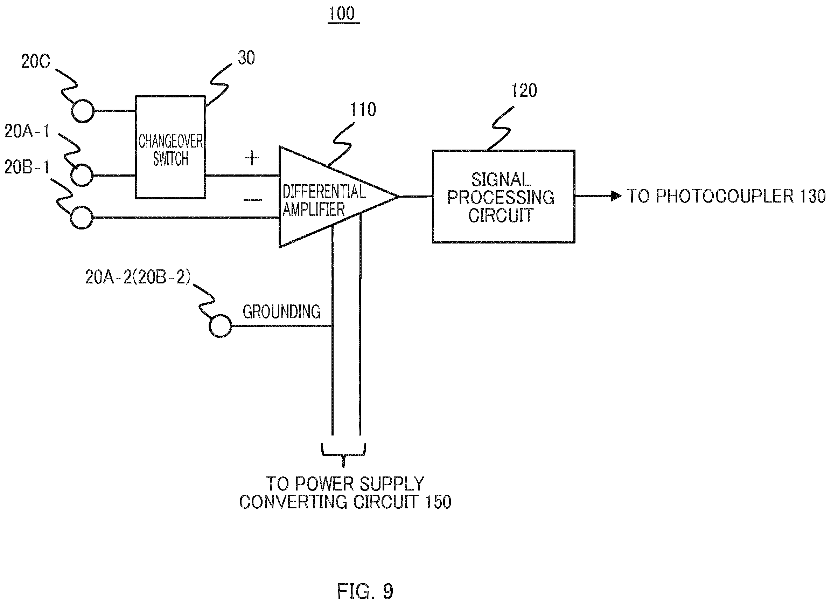

FIG. 9 illustrates a circuit block diagram near the measuring electrodes of the ECG measurement device 1 of FIG. 7. In the ECG measurement device 1 of the present modified example, the measuring electrode 20C provided on the end surface of the device is connected in parallel to the measuring electrode 20A-1. A changeover switch 30 is provided between the measuring electrodes 20A-1 and the measuring electrode 20C, and the differential amplifier 110, so that the input to the differential amplifier 110 can be switched between the measuring electrode 20A-1 and the measuring electrode 20C. As the changeover switch 30, a small push button switch or the like which is suitably attached to the ECG measurement device 1 may be used appropriately. It should be noted that if there is no functional problem as the ECG measurement device 1, the configuration is also applicable in which only the measuring electrode 20C is provided in parallel to the measuring electrode 20A-1 without additionally providing the changeover switch 30.

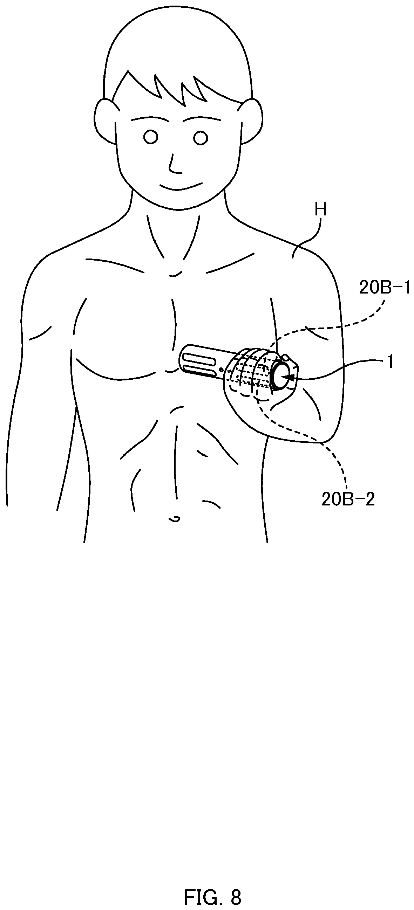

A description will be given regarding the ECG measurement method according to the present modified example. FIG. 8 schematically illustrates a usage state of the ECG measurement device 1 of the present modified example. It is assumed that a subject H has a problem with a function of the right hand or right arm and cannot hold the ECG measurement device 1 by gripping it with both hands. In this case, after the subject H turns on the power supply switch 12, the subject H grips the ECG measurement device 1 with his/her left hand and lifts it so as to bring the skin of the hand into contact with the measuring electrode 20B-1 and the ground electrode 20B-2. Then, the subject H presses the measuring electrode 20C provided on one end surface of the ECG measurement device 1 against his/her chest accordingly. In this state, the ECG measuring circuit 100 can acquire an ECG waveform of LEAD I of the subject H from the measuring electrode 20B-1 being in contact with the left hand of the subject H and the measuring electrode 20C being in contact with his/her chest. The processing contents of the signals in the ECG measuring circuit 100 are similar to the case of the embodiment in FIG. 1.

As described above, with the ECG measurement method according to the present modified example, even if the subject has a problem with the function of one hand or arm, or has lost them, simple and stable acquisition of an ECG waveform is possible. In this case, the measuring electrode 20B-1 is almost covered with the left hand gripping the device 1, and the measuring electrode 20C is positioned between the skin of the subject H and the case 10 of the ECG measurement device 1. This can give a shielding effect from external electromagnetic noise as in the previous embodiment.

In the present modified example, the description has been given of the configuration in which the measuring electrode 20C is additionally provided to the measuring electrode 20A-1. However, the ECG measurement device 1 having a specialized configuration for one-hand operation may also be applicable by providing the measuring electrode 20C instead of the measuring electrode 20A-1 and the ground electrode 20A-2.

### Other Modified Examples

The ECG measurement method of the present disclosure as described so far has been explained by an example realized using the cylindrical ECG measurement device 1. However, the present disclosure is not limited to such a configuration. In other words, it is sufficient that, when the ECG measurement is performed, the subject grips the measuring electrodes so as to wrap them, thus achieving a natural posture that does not cause a burden on muscles of the body such as unnecessary myoelectric signals to be a noise source. Accordingly, for example, it is also possible to provide a measuring electrode and a ground electrode at a part of a handrail having a substantially circular cross-section so as to simply and stably measure the ECG waveform of LEAD I. In this case, there can be provided an ECG waveform measurement means at a place such as a toilet, a bed, or an entrance of the house where the handrail for assistance has been installed.

As described above, the ECG signals of LEAD I can be stably measured with a simple configuration according to the ECG measurement method and the ECG measurement device of the present disclosure.

Although the present disclosure has been described with reference to example embodiments, those skilled in the art will recognize that various changes and modifications may be made in form and detail without departing from the spirit and scope of the claimed subject matter.

## Claims

1. An electrocardiographic measurement method, comprising:
preparing a bar body having a thickness capable of being gripped by a hand of a person who is a subject of an electrocardiographic measurement;
providing a first electrode and a second electrode for an electrocardiogram signal measurement of the subject with a space in an axial direction of the bar body and providing a ground electrode with a space in a circumferential direction of the bar body from at least one of the first electrode and the second electrode;
connecting an electrocardiogram measuring circuit between the first electrode and the second electrode, the electrocardiogram measuring circuit including an amplifier; and
measuring an electrocardiogram signal of the subject by the electrocardiogram measuring circuit through the first electrode and the second electrode while the subject grips the bar body at parts where right and left hands come to positions of the first electrode and the second electrode so as to bring the right and left hands of the subject into contact with the first electrode and the second electrode, respectively, and to bring the ground electrode into contact with one of the right and left hands.

2. The electrocardiographic measurement method according to claim 1, wherein
the first electrode and the second electrode, and the ground electrode paired with one of the first electrode and the second electrode with a space in a circumferential direction of the bar body are disposed at positions so as to be covered with the right and left hand of the subject.

3. The electrocardiographic measurement method according to claim 1, wherein
the bar body is a cylindrical case housing the electrocardiogram measuring circuit.

4. The electrocardiographic measurement method according to claim 1, wherein
the bar body is a part of a handrail on which a person puts a hand.

5. An electrocardiographic measurement method, comprising:
preparing a bar body having a thickness capable of being gripped by a hand of a person who is a subject of an electrocardiographic measurement;
providing a first electrode for an electrocardiogram signal measurement of the subject on a circumferential surface of the bar body and providing a ground electrode with a space in a circumferential direction of the bar body from the first electrode;
providing a second electrode for an electrocardiogram signal measurement of the subject on one end surface of the bar body in an axial direction;
connecting an electrocardiogram measuring circuit between the first electrode and the second electrode, the electrocardiogram measuring circuit including an amplifier; and
measuring an electrocardiogram signal of the subject by the electrocardiogram measuring circuit through the first electrode and the second electrode while the subject grips the bar body at a part where one hand comes to a position of the first electrode, and the second electrode is pressed against a chest near a heart of the subject so that the one hand of the subject gripping the bar body is brought into contact with the first electrode and the ground electrode, and the second electrode is brought into contact with the chest of the subject.

6. An electrocardiographic measurement device, comprising:
a bar body having a thickness capable of being gripped by a hand of a person who is a subject of an electrocardiographic measurement;
a first electrode and a second electrode for an electrocardiogram signal measurement of the subject, the first electrode and the second electrode being provided with a space in an axial direction of the bar body;
a ground electrode provided with a space in a circumferential direction of the bar body from at least one of the first electrode and the second electrode; and
an electrocardiogram measuring circuit connected between the first electrode and the second electrode, the electrocardiogram measuring circuit including an amplifier;
an electrocardiogram signal of the subject being measured by the electrocardiogram measuring circuit through the first electrode and the second electrode while the subject grips the bar body at parts where right and left hands come to positions of the first electrode and the second electrode so as to bring the right and left hands of the subject into contact with the first electrode and the second electrode, respectively, and to bring the ground electrode into contact with one of the right and left hands.

7. The electrocardiographic measurement device according to claim 6, wherein
the first electrode and the second electrode, and the ground electrode paired with one of the first electrode and the second electrode with a space in a circumferential direction of the bar body are disposed at positions so as to be covered with the right and left hand of the subject.

8. The electrocardiographic measurement device according to claim 6, wherein
the bar body is a cylindrical case housing the electrocardiogram measuring circuit.

9. The electrocardiographic measurement device according to claim 6, wherein
the bar body is a part of a handrail on which a person puts a hand.

10. An electrocardiographic measurement device, comprising:
a bar body having a thickness capable of being gripped by a hand of a person who is a subject of an electrocardiographic measurement;
a first electrode for an electrocardiogram signal measurement of the subject, the first electrode being provided on a circumferential surface of the bar body;
a ground electrode provided with a space in a circumferential direction of the bar body from the first electrode;
a second electrode for an electrocardiogram signal measurement of the subject, the second electrode being provided on one end surface of the bar body in an axial direction; and
an electrocardiogram measuring circuit connected between the first electrode and the second electrode, the electrocardiogram measuring circuit including an amplifier,
an electrocardiogram signal of the subject being measured by the electrocardiogram measuring circuit through the first electrode and the second electrode while the subject grips the bar body at a part where one hand comes to a position of the first electrode, and the second electrode is pressed against a chest near a heart of the subject so that the one hand of the subject gripping the bar body is brought into contact with the first electrode and the ground electrode, and the second electrode is brought into contact with the chest of the subject.
